Europäisches Patentamt

European Patent Office

Office européen des brevets

(11) Veröffentlichungsnummer : **0 048 987**
**B1**

(12) **EUROPÄISCHE PATENTSCHRIFT**

(45) Veröffentlichungstag der Patentschrift :
11.04.84

(51) Int. Cl.³ : **C 07 C 67/08, C 07 C 67/54**

(21) Anmeldenummer : 81107710.6

(22) Anmeldetag : 29.09.81

(54) Verfahren zur Herstellung eines Ethylesters.

(30) Priorität : 01.10.80 DE 3037158

(43) Veröffentlichungstag der Anmeldung :
07.04.82 Patentblatt 82/14

(45) Bekanntmachung des Hinweises auf die Patenterteilung : 11.04.84 Patentblatt 84/15

(84) Benannte Vertragsstaaten :
BE DE FR GB IT NL

(56) Entgegenhaltungen :
FR-A- 657 812
GB-A- 1 173 089
GB-A- 1 394 651

(73) Patentinhaber : HOECHST AKTIENGESELLSCHAFT
Postfach 80 03 20
D-6230 Frankfurt am Main 80 (DE)

(72) Erfinder : Leupold, Ernst Ingo, Dr.
Am Zäunefeld 15
D-6392 Neu-Anspach (DE)
Erfinder : Schmidt, Hans-Joachim, Dr.
Am Burgenblick 6
D-6240 Königstein/Taunus (DE)
Erfinder : Popp, Knut
Hasenpfad 11
D-6232 Bad Soden am Taunus (DE)

Verfahren zur Herstellung eines Ethylesters

Die vorliegende Erfindung betrifft ein Verfahren zur Herstellung eines Ethylesters aus ethanolhaltigen Gemischen aliphaster Alkohole.

Bei der CO-Hydrierung an heterogenen Katalysatoren gemäß den deutschen Offenlegungsschriften 2,628,463 und 2,748,097 entstehen u. a. Gemische aliphatischer Alkohole, insbesondere Ethanol und die verschiedenen Propanole und Butanole. Diese Alkoholgemische kann man von den anderen gasförmigen und flüssigen Produkten der CO-Hydrierung trennen und durch Veresterung mit einer Carbonsäure in ein Gemisch der entsprechenden Ester überführen, von denen der Ethylester, insbesondere (bei Verwendung von Essigsäure als Carbonsäure) der Essigsäureethylester, ein wichtiger Rohstoff für die Lackindustrie ist.

Bisher war kein Verfahren bekannt, wonach der Ethylester in einundderselben Kolonne gebildet und gleichzeitig von den übrigen Estern abgetrennt werden kann. Die bekannten Verfahren betreffen lediglich die Trennung eines Alkohols und einer Säure von dem aus ihnen gebildeten Ester (siehe etwa FR-A-657 812).

Es wurde nun ein Verfahren zur Herstellung eines Ethylesters aus ethanolhaltigen Gemischen aliphatischer Alkohole gefunden, das dadurch gekennzeichnet ist, daß man die Alkohole in Gegenwart eines sauren Katalysators mit einer Carbonsäure in einer Destillationskolonne verestert und kontinuierlich in derselben Kolonne aus der gebildeten Estermischung den Ethylester abtrennt, wobei man das Alkoholgemisch in den Sumpf und die Carbonsäure in das mittlere Drittel der Kolonne kontinuierlich einleitet, und wobei man den Ethylester sowie das bei der Veresterung entstehende Wasser am Kopf der Kolonne und die höheren Ester aus dem Sumpf kontinuierlich entnimmt.

Nach dem erfindungsgemäßen Verfahren lassen sich alle ethanolhaltigen Gemische aliphatischer Alkohole mi 2 bis 8 C-Atomen, die z. B. neben Ethanol die isomeren Propanole, Butanole, Pentanole und Hexanole sowie 2-Ethylhexanol enthalten können, unter gleichzeitiger Abtrennung des Ethylesters verstern. Besonders geeignet ist das Gemisch aus Ethanol und den isomeren Propanolen und Butanolen.

Die eingesetzten Gemische können neben den aliphatischen Alkoholen bereits Ester enthalten, ohne daß dadurch Störungen eintreten. So enthalten die nach DE-OS 2,628,463 entstehenden Reaktionsgemische Essigsäureethylester. Dieser durchläuft das erfindungsgemäße Verfahren ohne chemische Umwandlung, wenn Essigsäure als Carbonsäure eingesetzt wird. Andere Komponenten, wie zum Beispiel Acetaldehyd und Wasser, sollten vorher abgetrennt werden, da sie zu Nebenreaktionen bzw. zu mangelhaftem Umsatz führen können.

Zur Veresterung des Alkoholgemisches können im Prinzip alle organischen Carbonsäuren eingesetzt werden ; bevorzugt sind aliphatische Carbonsäuren mit 2 bis 8 C-Atomen, insbesondere Essigsäure.

Das Molverhältnis der Carbonsäure zur Summe der Alkohole kann in weiten Grenzen schwanken ; vorzugsweise beträgt es jedoch 1 : 1 bis 5 : 1, insbesondere 1,1 : 1 bis 2,0 : 1.

Als Katalysatoren werden vorzugsweise starke Säuren eingesetzt, insbesondere $H_2SO_4$ oder Ionenaustauschharze mit $SO_3H$-Gruppen.

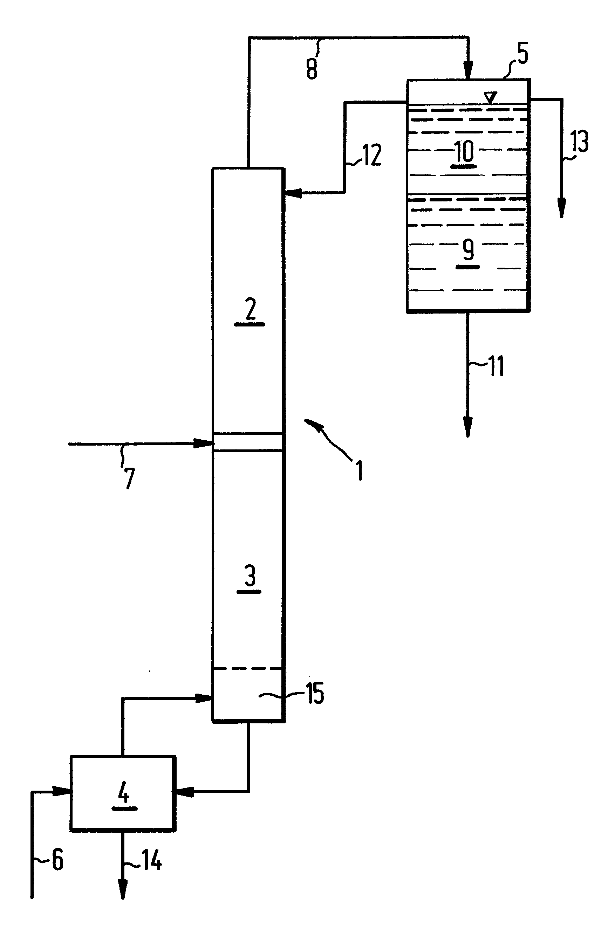

Eine bevorzugte Ausführungsform des erfindungsgemäßen Verfahrens ist in der Figur dargestellt. Die Apparatur besteht aus einer Kolonne (1) mit je 10 Glockenböden im Verstärkungsteil (2) und im Abtriebsteil (3), aus dem Verdampfer (4), sowie der Vorlage (5). Das Alkoholgemisch wird über Leitung (6) dem Verdampfer (4) zugeführt ; die Carbonsäure wird über Leitung (7) zwischen Verstärkungsteil (2) und Abtriebsteil (3) zugeführt. Das Kopfprodukt der Kolonne gelangt über Leitung (8) in die Vorlage (5) und teilt sich dort in zwei Phasen. Die untere (wäßrige) Phase (9) wird über Leitung (11) entnommen. Die obere (organische) Phase (10) ist im wesentlichen der gewünschte Ethylester. Dieser wird teils als Rücklauf über Leitung (12) in die Kolonne zurückgegeben, teils über Leitung (13) entnommen.

Die Carbonsäureester der höheren Alkohole werden dagegen zusammen mit überschüssiger Carbonsäure über Leitung (14) dem Verdampfer (4) entnommen.

Wird als Veresterungskatalysator eine flüssige starke Säure verwendet, so wird diese gelöst in der Carbonsäure über Leitung (7) in die Kolonne eingebracht.

Bei Verwendung eines heterogenen sauren Katalysators wird dieser als Schüttung in den unteren Teil (15) der Kolonne (1) eingebracht.

Beispiel 1

Es wird die in der Figur dargestellte Apparatur benutzt. Die Kolonne (1) mit jeweils 10 Glockenböden im Verstärkungsteil (2) und im Abtriebsteil (3) hat einen Innendurchmesser von 50 mm.

In den elektrisch beheizten Verdampfer (4) dosiert man bei einer Sumpftemperatur von 108 °C 50 g/h eines Gemischs aus 14,1 Gew.% Essigsäureethylester, 40,4 Gew.% Ethanol, 11,1 Gew.% n-Propanol, 11,2 Gew.% n-Butanol und 23,2 Gew.% $H_2O$.

Oberhalb des 10. Bodens der Kolonne pumpt man über Leitung (7) 50 g/h Essigsäure mit einen Anteil von 0,1 Gew.% $H_2SO_4$.

In der Vorlage (5) fällt eine obere, organische Phase (10) und eine untere, wässrige Phase (9) an. Der oberen Phase entnimmt man über Leitung (13) einen Anteil von 43,5 g/h mit einer Zusammensetzung von 96,4 Gew.% Ethylacetat, 0,6 Gew.% Ethanol, 3,0 Gew.% $H_2O$ und weniger als

0,01 Gew.% Essigsäure. Der Rest wird als Rücklauf über Leitung (12) verwendet ; das Rücklaufverhältnis ist 25. Das der oberen Phase (10) entnommene Produkt enthält über 90 % der theoretisch aus dem eingesetzten Ethanol herstellbaren Menge an Essigsäureethylester ; die untere Phase (9) enthält neben Wasser noch 7,7 Gew.% Essigsäureethylester und 3,8 Gew.% Ethanol. Die Kopftemperatur der Kolonne beträgt 71 °C.

Aus dem Verdampfer (4) entnimmt man kontinuierlich ein Gemisch aus 27,9 Gew.% Essigsäurenpropylester, 25,0 Gew.% Essigsäure-n-butylester und 45,0 Gew.% Essigsäure.

Beispiel 2

Es wird die in der Figur und in Beispiel 1 beschriebene Apparatur benutzt. Im unteren Teil (15) der Kolonne befinden sich 185 g eines handelsüblichen Kationenaustauschharzes mit $SO_3H$-Gruppen.

In den Verdampfer (4) dosiert man bei einer Sumpftemperatur von 115 °C 55 g/h einer Mischung aus 40,2 Gew.% Ethanol, 11,5 Gew.% n-Propanol, 11,5 Gew.% n-Butanol und 36,8 Gew.% $H_2O$.

Oberhalb des 10. Bodens der Kolonne dosiert man über Leitung (7) 50 g/h Essigsäure.

Bei einer Kopftemperatur von 71 °C besteht die obere, organische Phase (10) des Destillats aus 97,0 Gew.% Essigsäureethylester, 2,5 Gew.% $H_2O$, 0,5 Gew.% Ethanol und weniger als 0,01 Gew.% Essigsäure. Das Rücklaufverhältnis beträgt 25.

Die wässrige Phase (9) enthält 6,9 Gew.% Essigsäureethylester und 2,3 Gew.% Ethanol.

Das über Leitung (14) entnommene Sumpfprodukt enthält 36,1 Gew.% Essigsäure, 22,0 Gew.% Essigsäure-n-propylester und 36,5 Gew.% Essigsäure-n-butylester.

**Anspruch**

Verfahren zur Herstellung eines Ethylesters aus ethanolhaltigen Gemischen aliphatischer Alkohole, dadurch gekennzeichnet, daß man die Alkohole in Gegenwart eines sauren Katalysators mit einer Carbonsäure in einer Destillationskolonne verestert und kontinuierlich in derselben Kolonne aus der gebildeten Estermischung den Ethylester abtrennt, wobei man das Alkoholgemisch in den Sumpf und die Carbonsäure in das mittlere Drittel der Kolonne kontinuierlich einleitet, und wobei man den Ethylester sowie das bei der Veresterung entstehende Wasser am Kopf der Kolonne und die höheren Ester aus dem Sumpf kontinuierlich entnimmt.

**Claim**

A process for the preparation of an ethyl ester from ethanol-containing mixtures of aliphatic alcohols, characterized by esterifying the alcohols with a carboxylic acid in the presence of an acid catalyst in a distilling column and separating in the same column the ethyl ester continuously from the ester mixture obtained, the alcohol mixture being fed to the column in the sump and the carboxylic acid being fed in at a point located in the central part and the ethyl ester and the water formed during esterification being withdrawn continuously at the top of the column and the higher esters being withdrawn continuously at the sump.

**Revendication**

Procédé de préparation d'un ester éthylique à partir de mélanges d'alcools aliphatiques contenant de l'éthanol, procédé caractérisé en ce qu'on estérifie les alcools en présence d'un catalyseur acide à l'aide d'un acide carboxylique dans une colonne de distillation et l'on sépare continuellement dans la même colonne, à partir du mélange des esters formés, l'ester éthylique, en introduisant continuellement le mélange des alcools dans le réservoir et l'acide carboxylique dans le tiers moyen de la colonne, et en retirant continuellement l'ester éthylique ainsi que l'eau résultant de l'estérirification en tête de la colonne et les esters supérieurs du réservoir de pied de colonne.